# EUROPÄISCHE PATENTSCHRIFT

(11) **EP 0 999 753 B1**
(45) Veröffentlichungstag und Bekanntmachung des Hinweises auf die Patenterteilung: **09.05.2001**
(21) Anmeldenummer: 98946228.8
(22) Anmeldetag: 04.07.1998
(51) Int. Cl.: A22B 5/00, G01N 33/12

(54) **VERFAHREN ZUR BEWERTUNG VON SCHLACHTTIERHÄLFTEN DURCH OPTISCHE BILDVERARBEITUNG**
METHOD FOR EVALUATING HALF-CARCASSES BY MEANS OF OPTICAL IMAGE PROCESSING
PROCEDE POUR EVALUER PAR TRAITEMENT D'IMAGE OPTIQUE DES MOITIES D'ANIMAUX ABATTUS

(30) Priorität: 01.08.1997 DE 19733216
(43) Veröffentlichungstag der Anmeldung: 17.05.2000
(73) Patentinhaber: CSB-System Software-Entwicklung & Unternehmensberatung AG, 52511 Geilenkirchen (DE)
(72) Erfinder: SCHIMITZEK, Peter, D-52511 Geilenkirchen (DE)
(74) Vertreter: Haussingen, Peter
(86) Internationale Anmeldenummer: DE9801926
(87) Internationale Veröffentlichungsnummer: WO9905916

(56) Entgegenhaltungen:
- WO-A-93/21597
- DE-A- 4 109 345
- DE-A- 4 408 604

## Beschreibung

Die Erfindung bezeichnet ein Verfahren zur Bewertung von Schlachttierhälften durch optische Bildverarbeitung, die am Wareneingang, Klassifizierungspunkten oder Warenausgang von Schlacht- und Fleischwarenbetrieben erfaßt werden. Derartige Schlachttierhälften werden in der Regel an einem Hacken hängend mittels spezieller Transportsysteme durch die Schlacht- und Fleischwarenbetriebe befördert. Das vorgestellte Verfahren ist insbesondere für die Bewertung von Schlachtschweinehälften ausgelegt, jedoch prinzipiell ebenfalls für Schlachttierhälften von Rindern, Schafen, Ziegen oder andere Groß- und Kleinschlachttieren geeignet.

Im allgemeinen werden die Schlachtschweinehälfte registriert, gewogen und bewertet. Die wirtschaftliche Bewertung der Schlachtschweinehälften erfolgt durch eine entsprechende amtliche Handelsklasseneinstufung. Danach erfolgt die Erfassung der Speck - und Fleischdicken jeweils landesspezifisch an gesetzlich vorgegebenen Stellen. Zur Sortierung erfolgt in der Regel eine Handelswertbestimmung der Schlachtkörper mit einem höheren Aussagegehalt durch die Einbeziehung einer Vielzahl weiterer Parameter, welche jedoch meist nicht standardisiert sind.

Die Druckschrift DE OS 2728913 beschreibt allgemein die Verwendung von optischen/mechanischen Sensoren zur Erfassung von Kennwerten des Speck-/Fleischanteils von Schlachttierhälften an Transporteinrichtungen und die automatische und selbständige Verarbeitung dieser Werte durch einen Rechner zur Klassifizierung von Fleisch. Dazu wird die Schlachttierhälfte, insbesondere eine Schlachtschweinehälfte, in einer Dunkelkammer optisch abgetastet, der Schinken mit Mitteln der Bildanalyse erfaßt und bewertet. Das ermittelte Klassifizierungsmerkmal wird anschließend über einen Laser auf die Schlachttierhälfte aufgebracht. Das aufgezeigte Verfahren basiert hauptsächlich auf der Bestimmung von Parametern der Schinkenregion und ist zu aufwendig, um in Betrieben jeder Größenordnung eingesetzt werden zu können.

Eine Methode zur Handelsklasseneinstufung ist nach der Druckschrift DE 1673038 die Messung der Speck- und Fleischdicke mit einer Nadel über elektro-optische Verfahren an gesetzlich vorbestimmten Meßstellen der Schlachtschweinehälften. Dabei wird eine optische Nadel an vorbestimmter Meßstelle in das Fleisch jeweils einer Schlachtschweinehälfte eingestochen, die an der Meßstelle optisch die Dicke des Fleisches (Muskel) und des Specks zu bestimmen gestattet. Für die Durchführung der Messung ist in der Regel eine Arbeitskraft nötig. Nachteile dieser Vorgehensweise sind hygienische Bedenklichkeit des Einstiches, die hohen Investitionskosten für die spezielle optische Nadel, die starke subjektive Abhängigkeit der Meßergebnisse durch die Positionierung der Nadel und den Winkel des Einstiches und die fehlende Transparenz der Messung durch die andere Partei, da es sich um ein inneres Meßverfahren handelt. Durch das Ein-Punkt-Verfahren ist die Aussagefähigkeit prinzipiell gering, so daß eine mangelnde Aussagekraft über den tatsächlichen Handelswert des Schlachtschweines vorliegt.

Zur Handelsklasseneinstufung von Schlachtschweinen ist seit den 70iger Jahren das ZP(Zweipunkte)-Verfahren mit Messungen ausschließlich im Lendenbereich eine andere gängige Vorgehensweise. Dabei werden an zwei markanten und leicht identifizierbaren gesetzlich vorgegebenen Punkten Längenmessungen vorgenommen. Die Messungen können manuell mit einem Lineal oder alternativ mit einem Meßschieber, welche teilweise online mit einem Rechner zur Datenerfassung in Verbindung stehen, vorgenommen werden. Mit diesen Werten wird über eine Berechnungsvorschrift (zur Zeit eine quadratische Gleichung mit den zwei gemessenen Längen als Variable) die Klassifizierung ermittelt. Die Vorteile des ZP-Verfahrens liegen insbesondere bei den geringen Investitionskosten, leichter Erlernbarkeit und der großen Transparenz der ermittelten Werte. Dadurch wird dieses Verfahren selbst in sehr kleinen Schlachtbetrieben eingesetzt. Durch die Verwendung von 2 günstigen Meßstellen, läßt sich der tatsächliche Handelswert prinzipiell exakter als mit einem Ein-Punkt-Verfahren bestimmen. Nachteile des ZP-Verfahrens sind die manuell aufwendige Messung an zwei Stellen, eine geringe zeitliche Einstufungsleistung sowie die subjektive Abhängigkeit der Ergebnisse.

In der Druckschrift EP 0029562 wird ein Verfahren zur Handelsklasseneinstufung beschrieben, bei welcher in der Schinkenregion der Schinkenwinkel automatisch mechanisch bestimmt wird. In den Weiterbildungen wird zu dessen Bestimmung ein videooptisches Gerät zur Aufnahme eines Bildes der Schlachtschweinehälfte verwendet, welches den Schinkenwinkel und optional zusätzlich das Maß der minimalen Speckdicke über den MGM(Musculus-Gluteus-Medius) im Lendenbereich durch den Einsatz der Rechentechnik ermittelt Durch den Einsatz der Rechentechnik kann auf die manuelle Bewertung verzichtet werden und der subjektive Einfluß entfällt. Es handelt sich bei dieser Druckschrift um die Anwendung eines video-optischen Verfahrens zur Bestimmung von Konturen, deren Parameter zur Handelsklasseneinstufung verwendet werden. Nachteilig ist jedoch, daß das beschriebene Verfahren hauptsächlich auf der Bestimmung von Parametern der Schinkenregion beruht, welche mit erfaßt und ausgewertet werden muß.

In der Druckschrift DD 259346 wird ein einfaches Verfahren zur Klassifizierung von Schlachttierhälften beschrieben, bei welcher eine Zeilenkamera, welche die Lendenregion abfährt, die reflektierten Hell-Dunkelwerte registriert und bewertet. Es gelingt dadurch, den Speck- und Fleischanteil näherungsweise zu bestimmen. Nachteilig ist, daß dieses Verfahren nicht dem erforderlichen Standard zur Klassifizierung entspricht.

In der Druckschrift DE 4131556 wird ein Verfahren beschrieben, bei welchem mit Bildverarbeitungsverfahren auf der Basis einer video-optischen Aufnahme einer Schlachttierhälfte die Lage von inneren Organen durch eine Objektanalyse mit analytischen Modellorganen des Skeletts, insbesondere der Wirbelsäule und der Wirbel, bestimmt wird. Derartige rechenintensive Verfahren sind notwendig, um nachgeschaltete automatische Zerlegemaschinen für Schlachttierhälften präzis steuern zu können. Eine Bewertung von Fleisch-Rückenspeckverhältnissen erfolgt zusätzlich. Es erfolgt ebenfalls eine Analyse des Schinkenbereiches. Für eine Bewertung von Schlachttierhälften zu Klassifizierung ist eine Erfassung des Skeletts unnötig somit das Verfahren damit unnötig kompliziert und in der praktischen Umsetzung zu teuer.

In der Druckschrift WO-A-9321597 wird ein Verfahren zur Bewertung von Schlachttierhälften beschrieben, bei welchem ein einzelnes, durch einen optischen Sensor aufgenommenes Farbbild von Teilen einer Schlachttierhälfte EDV-gestützt vollautomatisch ausgewertet wird. Nachteil der Lösung ist es, daß hier kein Meßverfahren auf Basis standardisierter Bewertungsmethoden, die Handelsklasseneinstufung schnell, objektiv, transparent und in Schlacht- und Fleischwarenbetrieben jeder Größenordnung einheitlich vornehmbar, vorliegt.

Die Aufgabe der Erfindung ist die Beseitigung bestehender Nachteile des Standes der Technik und die Entwicklung eines automatischen Bewertungsverfahrens für Schlachttierhälften, welches durch berührungslose Meßverfahren auf Basis standardisierter Bewertungsmethoden die Handelsklasseneinstufung schnell, objektiv, transparent und in Schlacht- und Fleischwarenbetrieben jeder Größenordnung einheitlich vorzunehmen gestattet. Dazu muß das Verfahren auf einer standardisierten Bewertungsmethode basieren, die zuverlässige Ergebnisse zur Handelsklasseneinstufung liefert und einfach nachprüfbar ist. Das Meßverfahren muß mit einfachen Mitteln und geringen Investitionskosten die Verwendung in Kleinstbetrieben ermöglichen und dennoch die Bewertungsleistung von Großbetrieben gewährleisten. Dazu ist es zwingend erforderlich, eine Handelsklasseneinstufung und/oder Handelswertermittlung so einfach wie möglich und nur so genau wie notwendig auszuführen. Insbesondere soll sich auf eine von außen zugängliche charakteristische Körperregion des Schlachttiers beschränkt werden. Es ist günstig, die Handelsklasseneinstufung optional mit einer gleichzeitigen Handelswertermittlung zu kombinieren.

Die Aufgabe wird mit den im Patentanspruch 1 genannten Merkmalen gelöst. Weiterbildungen der Erfindung ergeben sich aus den Unteransprüchen.

Durch die Bestimmung der Handelsklasseneinstufung auf Basis von mittels der optischen Bildauswertung zugänglichen äußeren Parametern in der Lenden - und Schinkenregion erfolgt in Anlehnung an das ZP-Verfahren eine zuverlässige und der Definition der Handelsklasseneinstufung folgende Bewertung von Schlachttierkörpern, ohne daß das Bewertungsverfahren durch Analyse weiterer Körperregionen und damit verbundener zusätzlich zu berücksichtigender Freiheitsgrade unnötig kompliziert und teuer in der praktischen Realisierung wird.

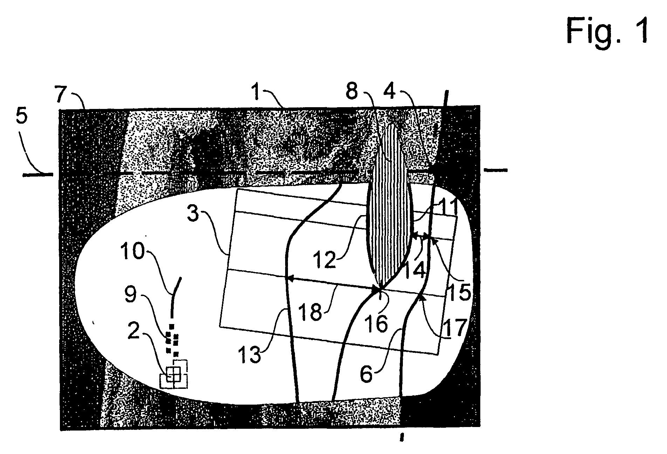

Die Erfindung wird nachstehend an Hand von
Fig. 1 als Bewertungsprinzip bei einer Schlachtschweinehälfte näher erläutert.

Wie in Fig. 1 aufgezeigt, stellt sich das Bewertungsprinzip bei einer Schlachtschweinehälfte wie folgt dar. Eine vorzugsweise an einem Transportband hängende Schlachtschweinehälfte ist zu bewerten, wenn sie einen Schlacht- und Fleischwarenbetrieben im Wareneingang, an Klassifizierungspunkten, oder im Warenausgang passiert. Je nach Art des Schlacht- oder Fleischwarenbetriebes befinden sich die Schlachtschweinehälften noch im warmen oder bereits im kalten Zustand bzw. in einem Zwischenzustand. Zur Verrechnung mit Dritten ist die zuverlässige und transparente Handelsklasseneinstufung von Bedeutung, die nach standardisierten Methoden zu ermitteln ist. Es ist weiterhin vorteilhaft, im Bereich der Kontroll-Punkte die Schlachtschweinehälfte zu wiegen und zu kennzeichnen, bzw. die Kennzeichnung zu registrieren. Zur Einbindung in ein übergeordnetes System zur Speicherung und Protokollierung sowie zur Fernwartung der Meß- und Auswertetechnik ist die Einbindung in ein übergeordnetes Kommunikationsnetz, bsw. Intranet, ISDN, Internet günstig.
In einem 1. Verfahrensschritt wird die Schlachtschweinehälfte (im Regelfall je eine pro Schlachttier), mit der Spaltseite an einer elektronischen Kamera, bsw. einem digitalen Photoapparat, einer Videokamera oder einem anderen optischen Sensor, vorbei geführt und ein Bereich, der stets die erweiterte Lendenregion beinhaltet, bsw. 30 x 40 cm, als Farbbild 1 mit hinreichender Auflösung (mindestens 800x600) digital aufgenommen und online an die EDV übertragen. Das optische Farbbild 1 von Teilen einer Schlachtschweinehälfte wird nachfolgend EDV-gestützt vollautomatisch ausgewertet. Es wird ausschließlich die von außen optisch zugängliche erweiterte Lendenregion der Schlachttierhälfte zur Bewertung verwendet. Dazu wird das Farbbild 1 in Echtzeit in den Rechner eingelesen und entsprechend geeignet gespeichert, bsw. als je ein Bitmap für das Rot-, Grün- und Blaubild in ausreichender Quantisierung (mindestens 8 Bit). Aus diesen Bitmaps können einfach geeignete analytische Terme je Bildpunkt berechnet werden, welche die Farb- und Helligkeitsinformation beinhalten. Durch Bewertung von über gleichartigen Bildbereichen 2 berechneten Ausdrücken dieser Farb- und Helligkeitsinformationen lassen sich bestimmte Bildbereiche 2 oder denen zugeordnete Pixel selektieren und bsw. über ein Status-Bitmap im Rechner indizieren.
In einem 2. Verfahrensschritt wird der Auswertebereich 3 als Teilbild der erweiterten Lendenregion der Schlachtschweinehälfte für die nachfolgenden Verfahrensschritte in einer geeigneten Größe selektiert um den Rechenaufwand und den Speicherplatzbedarf zu verringern. Dies kann durch Selektion eines Ursprungspunktes 4 in Verbindung mit dem relativ dazu positionierten Auswertebereich 3 erfolgen, bsw. durch Selektion eines besonders hellen Bildstreifens 5 als Extremwert im Helligkeitssignal, welcher den hohen Fettanteil am hinteren Ende der Schlachtschweinehälfte selektiert, und dessen rükkenseitiger äußerer Konturgrenze 6 des Schlachttierkörpers vor dem Bildhintergrund 7, der günstig einfarbig gewählt wird, zu dem sich der Auswertebereich 3 von etwa 10 x 15 cm positioniert, welcher insbesondere den MGM(Musculus-Gluteus-Medius) 8 beinhaltet. Nach erfolgter Selektion des Teilbildes können die übrigen Bildinformationen aus dem Speicher entfernt werden. Optional kann das Teilbild vollständig oder verdichtet an zentraler Stelle zur Protokollierung abgespeichert werden.
Im 3. Verfahrensschritt wird der gesamte Auswertebereich gleichartig nach geeigneten Unterschieden zwischen benachbarten Bildbereichen analysiert. Die Bildbereiche können dabei aus mehreren benachbarten Pixeln bestehen, die mit unterschiedlicher Gewichtsfunktion bewertet werden, wodurch Fuzzy basierende Methoden angewendet werden können. Zur Selektion wesentlicher Unterschiede werden wiederum geeignete analytische Terme aus den Farb- und Helligkeitsinformation gebildet, die im gegenseitigen Bezug verglichen werden. Bei hinreichenden Unterschieden, deren Aussagegehalt zusätzlich über ein aus der inneren Streuung der Bildbereiche gebildetes Vertrauensmaß gewichtet werden kann, erfolgt die punktuelle Zuordnung zu einem Konturpunkt 9, der über eine entsprechende Indizierung im Pixelbereich bsw. im Status-Bitmap als Konturbildbereich gespeichert wird und optional mit einem Vertrauensmaß gewichtet ist.

Im 4. Verfahrensschritt erfolgt durch Verfahren der Kantenglättung die Zuordnung von mathematisch glatten Linienkonturen 10 zu den Konturbildbereichen. Dies kann bsw. durch eine Spline-Anpassung bei metrischer Norm erfolgen, die optional mit dem Vertrauensmaß gewichtet werden kann. Die Linienkontur 10 wird ebenfalls im Status-Bitmap gespeichert und kann ein Vertrauensmaß beinhalten. Unterschreitet das Vertrauensmaß eine untere Schranke, gilt die Linienkontur 10 als beendet.
Im 5. Verfahrensschritt wird bsw. über eine Abzählung der erfaßten Linienkonturen 10 von der rückenseitigen äußeren Konturgrenze 6 des Schlachtschweinekörpers kommend die Speck/MGM-Grenze 11, die MGM/Fettgrenze 12 und der Knochenmarkkanal 13 den Linienkonturen 10 zugeordnet und gespeichert. Es ist möglich, für die einzelnen Konturen jeweils Plausibilitätsgrenzen zuzuordnen.
Im 6. Verfahrensschritt erfolgt entsprechend der Linienkonturen 10 eine Klassifikation der Schlachtschweinehälfte nach der ZP(Zweipunkt)-Methode. Dazu wird durch eine Berechnungsroutine die minimale Strecke zwischen der rückenseitigen äußeren Konturgrenze 6 und der Speck/MGM-Grenze 11 selektiert, welche der minimalen Speckdicke 14 entspricht, und deren Schnittpunkt mit der äußeren Konturgrenze als 1. Punkt des ZP-Verfahrens 15 festgelegt. Von diesem Punkt ausgehend wird die Linienkontur 10 der Speck/MGM-Grenze 11 und die der MGM/Fettgrenze 12 verfolgt, bis sie sich in einem Schnittpunkt 16 schneiden. In dem Fall, daß die MGM/Fettgrenze 12 zuvor endet, kann sie optional spitzwinklig zur Speck/MGM-Grenze 11 verlängert werden. Das Lot auf die rückenseitige äußere Kontur 6 bildet den 2. Punkt des ZP-Verfahrens 17 und gestattet in entgegengesetzter Richtung die Bestimmung der Muskeldicke 18 als Strecke vom Schnittpunkt 16 bis zum Knochenmarkkanal 13. Damit sind die beiden von der derzeit standardisierten ZP-Methode benötigten Meßwerte automatisch erfaßt und können in der zugeordneten Berechnungsroutine zu einem Bewertungsmaß verarbeitet und zentral gespeichert werden. Es besteht natürlich die Möglichkeit, gleicherart als weitere Strecken die Speck- und Fleischmaße als Meßwerte jeweils an beiden selektierten Meßpunkten zu erfassen, welche bsw. bei weiterentwickelten bzw. modifizierten ZP-Methoden eine standardisierte Anwendung finden können.

In den Weiterbildungen der Erfindung ist die Kombination einer standardisierten Bewertung, wie zur Handelsklasseneinstufung nach der ZP-Methode bzw. deren Weiterentwicklungen notwendig mit zusätzlichen Bewertungsverfahren denkbar. Derartige Bewertungsverfahren gestatten durch die Flexibilität ihrer Modifizierung und der Ausnutzung der Möglichkeiten moderner rechnergestützter Verfahren prinzipiell einen höheren Aussagegehalt über den tatsächlichen Handelswert und einer genaueren Handelsklasseneinstufung, welche bsw. zur besseren und genaueren Beurteilung der Schlachtkörper verwendbar sind. Dazu bietet sich eine geeignete Kombination von Kennwerten an, die sich als Parameter aus den erfaßten Konturen des Schlachttierkörpers, der ebenfalls vorhandenen Farbinformation sowie einer zusätzlich erfaßten Masse ergeben. Denkbar sind die Vermessung zusätzlicher Strecken an festgelegten Stützpunkten oder die Bestimmung von Krümmungen. In geschlossenen Konturen lassen sich Flächenmaße bestimmen. Auf Basis der Farbinformation kann eine Korrelation zur Fleischqualität hergestellt werden, indem das Fleisch in verschiedene Qualitätsstufen eingeteilt wird, bsw. in Normal, PSE (pail-soft-exudative) und DFD(dark firm dry) sowie weitere Merkmale, z.B. eine verstärkte Fütterung mit Mais, erkennbar sind. Die zugehörige Bewertungsvorschrift ist, da nicht notwendig standardisiert, ständig optimierbar, was an Hand von Referenzuntersuchungen zum tatsächlichen Handelswert, durch Vergleich mit anderen Bewertungsverfahren usw. möglich ist. Durch den Einsatz von Methoden der KI(Künstliche Intelligenz) läßt sich diese Optimierungsarbeit an zentraler Stelle automatisieren. Über eine Remote-Steuerung läßt sich eine neue optimierte Bewertungsvorschrift als Update am Stichtag auf alle Nutzer, auch an räumlich entfernten Orten, einfach übertragen. Ebenso ist (falls erforderlich) zur Kontrolle das Auslesen ebendieser Bewertungsvorschrift und weiterer Kennwerte vorstellbar. Falls der durch die Bildaufnahme erfaßte Bereich eine Kennzeichnung zur Identifizierung des Schlachtschweines beinhaltet, kann diese in einer zugeordneten Routine erfaßt, gelesen und gespeichert werden.

### verwendete Bezugszeichen

- 1: Farbbild
- 2: Bildbereich
- 3: Auswertebereich
- 4: Ursprungspunkt
- 5: heller Bildstreifen
- 6: rückenseitige äußere Konturgrenze
- 7: Bildhintergrund
- 8: MGM
- 9: Konturpunkt
- 10: Linienkonturen
- 11: Speck/MGM-Grenze
- 12: MGM/Fettgrenze
- 13: Knochenmarkkanal
- 14: minimale Speckdicke
- 15: 1. Punkt des ZP-Verfahrens
- 16: Schnittpunkt
- 17: 2. Punkt des ZP-Verfahrens
- 18: Muskeldicke

## Patentansprüche

1. Verfahren zur Bewertung von Schlachttierhälften durch optische Bildverarbeitung, bei welchem ein einzelnes, durch einen optischen Sensor aufgenommenes optisches Farbbild (1) von Teilen einer Schlachttierhälfte EDV-gestützt vollautomatisch ausgewertet wird, dadurch gekennzeichnet,
daß ausschließlich die von außen optisch zugängliche erweiterte Lendenregion der Schlachttierhälfte zur Bewertung verwendet wird,
daß aus den einzelnen Farbsignalen der Pixel gebildete Terme je Bildbereich (2) gleichartig berechnet werden,
wobei im 1. Verfahrensschritt das Teilbild die erweiterte Lendenregion der Schlachtschweinehälfte erfaßt und elektronisch gespeichert wird,
im 2. Verfahrensschritt der Auswertebereich (3) nach der Analyse von berechneten Termen je Bildbereich (2) über dem Teilbild festgelegt wird,
im 3. Verfahrensschritt Konturpunkte (9) durch Analyse der Extremwerte von Differenzen von berechneten Termen je Bildbereich (2) über dem Auswertebereich (3) bestimmt und gespeichert werden,
im 4. Verfahrensschritt Linienkonturen (4) durch Kantenglättung von Konturbildbereichen berechnet und gespeichert werden,
im 5. Verfahrensschritt die rückenseitige äußere Konturgrenze (6), die Speck/MGM-Grenze (11), die MGM/Fettgrenze (12) und der Knochenmarkkanal (13) den Linienkonturen (10) zugeordnet und gespeichert werden,
im 6. Verfahrensschritt entsprechend der Linienkonturen (10) eine Klassifikation der Schlachttierhälfte nach der ZP-Methode erfolgt, indem am 1. Punkt des ZP-Verfahrens (15) die minimale Speckdicke (14) und am 2. Punkt des ZP-Verfahrens (17), d.h. im Schnittpunkt (16) der Speck/MGM-Grenze (11) mit der MGM/Fettgrenze (12), die Muskeldicke (18) bis zum Knochenmarkkanal (13) berechnet wird.

2. Verfahren zur Bewertung von Schlachttierhälften durch optische Bildverarbeitung nach Anspruch 1, dadurch gekennzeichnet,
daß eine sichere Bewertung im gesamten Temperaturbereich von warmen bis kalten Schlachttierkörpern erfolgen kann.

3. Verfahren zur Bewertung von Schlachttierhälften durch optische Bildverarbeitung nach einem der Ansprüche 1 bis 2, dadurch gekennzeichnet,
daß mit dem speziell dafür standardisierten ZP-Verfahren eine Bewertung an Schlachtschweinehälften vorgenommen wird.

4. Verfahren zur Bewertung von Schlachttierhälften durch optische Bildverarbeitung nach einem der Ansprüche 1 bis 3, dadurch gekennzeichnet,
daß die Schrittweite der Bildbereiche (2) kleiner oder gleich der zugehörigen Breite ist und die in den Bildbereichen enthaltenen Pixel mit unterschiedlichem Gewicht in die Berechnung eingehen.

5. Verfahren zur Bewertung von Schlachttierhälften durch optische Bildverarbeitung nach einem der Ansprüche 1 bis 4, dadurch gekennzeichnet,
daß aus der Varianz der Terme innerhalb der Bildbereiche (2) ein Vertrauensmaß bestimmt wird, welches bei weiteren Berechnungen den Bildbereichen (2) zugeordnet wird.

6. Verfahren zur Bewertung von Schlachttierhälften durch optische Bildverarbeitung nach einem der Ansprüche 1 bis 5, dadurch gekennzeichnet,
daß optional im 6. Verfahrensschritt der 2. Punkt des ZP-Verfahrens (17), d.h. der Schnittpunkt (16) mit der Speck/MGM-Grenze (11), über die spitzwinklige Projektion des Endpunktes der MGM/Fettgrenze (12) auf die Speck/MGM-Grenze (11)berechnet wird.

7. Verfahren zur Bewertung von Schlachttierhälften durch optische Bildverarbeitung nach einem der Ansprüche 1 bis 6, dadurch gekennzeichnet,
daß im 6. Verfahrensschritt die Weiterentwicklungen und Modifizierungen des ZP-Verfahrens und der Berechnungsvorschrift berücksichtigt sind.

8. Verfahren zur Bewertung von Schlachttierhälften durch optische Bildverarbeitung nach einem der Ansprüche 1 bis 7, dadurch gekennzeichnet,
daß im 6. Verfahrensschritt die Speck- und Fleischdicke an beiden ZP-Punkten bestimmt wird und in die Berechnung eingeht.

9. Verfahren zur Bewertung von Schlachttierhälften durch optische Bildverarbeitung nach einem der Ansprüche 1 bis 8, dadurch gekennzeichnet,
daß die Speck- und Fleischdicke an vorbestimmten Stützpunkten zwischen den ZP-Punkten bestimmt wird und in die Berechnung eingeht.

10. Verfahren zur Bewertung von Schlachttierhälften durch optische Bildverarbeitung nach einem der Ansprüche 1 bis 9, dadurch gekennzeichnet,
daß zu den Linienkonturen (10) charakteristische Kurvenparameter berechnet und gespeichert werden.

11. Verfahren zur Bewertung von Schlachttierhälften durch optische Bildverarbeitung nach Anspruch 10, dadurch gekennzeichnet,
daß über eine Bewertungsfunktion anhand der Kurvenparameter, der in bestimmten Bildbereichen (2) berechneten Fleischfarbe und einer zusätzlichen Gewichtsangabe eine Bewertung der Schlachttierhälften erfolgt und gespeichert wird.

12. Verfahren zur Bewertung von Schlachttierhälften durch optische Bildverarbeitung nach Anspruch 10, dadurch gekennzeichnet,
daß die Bewertungsfunktion durch Referenzmessungen und/oder KI-Systeme verifiziert ist.

13. Verfahren zur Bewertung von Schlachttierhälften durch optische Bildverarbeitung nach einem der Ansprüche 1 bis 12, dadurch gekennzeichnet,
daß im aufgenommenen Farbbild (1) zusätzlich enthaltene Identifizierungs-Informationen der Schlachttierhälfte selektiert, ausgelesen und gespeichert werden.

14. Verfahren zur Bewertung von Schlachttierhälften durch optische Bildverarbeitung nach einem der Ansprüche 1 bis 13, dadurch gekennzeichnet,
daß mittels Datenübertragung die Speicherung an einer zentralen Stelle erfolgt.

15. Verfahren zur Bewertung von Schlachttierhälften durch optische Bildverarbeitung nach Anspruch 14, dadurch gekennzeichnet,
daß die Bewertungsfunktion und Grundeinstellungen über eine Remote-Steuerung überwachbar und/oder updatebar ist.

## Claims

1. Method for the evaluation of sides of meat by visual image processing wherein a single, visual, colour image (1) of parts of a side of meat recorded by means of a visual sensor is subjected to a fully automatic, computer-assisted evaluation, characterised in that,
exclusively the externally visible, extended loin region of the side of meat is used for the evaluation,
that terms formed from the individual colour signals of the pixels are calculated for each image area (2) in the same manner,
whereby in the first procedural stage, the sub-image registers and stores electronically the extended loin region of the side of pork,
in the second procedural stage, the evaluation area (3) is determined via the sub-image after analysis of calculated terms for each image area (2),
in the third procedural stage, contour points (9) are determined and stored via the evaluation area (3) by analysis of the extreme values for differences from calculated terms in each image area (2),
in the fourth procedural stage, linear contours (4) are calculated by rounding the edges of contour image areas and placed in a memory,
in the fifth procedural stage, the rear, outermost contour boundary (6), the bacon/MGM [middle gluteus muscle] boundary (11), the MGM/fat boundary (12) and the bone-marrow channel (13) are allocated to the linear contours (10) and placed in a memory,
in the sixth procedural stage, the side of meat is classified with reference to the linear contours (10) in accordance with the two-point method, by calculating the minimum bacon thickness (14) at the first of the two reference points(15) and the muscle thickness (18) up to the bone-marrow channel (13) at the second of the two reference points (17), i.e. at the intersection (16) of the bacon/MGM boundary (11) with the MGM/fat boundary (12).

2. Method for evaluating sides of meat by visual image processing in accordance with Claim 1, characterised in that,
a reliable evaluation can be made over the whole temperature range from warm to cold sides of meat.

3. Method for evaluating sides of meat by visual image processing in accordance with any one of Claims 1 to 2, characterised in that,
an evaluation of sides of pork is carried out using the two-point-method specially standardised for this purpose.

4. Method for evaluating sides of meat by visual image processing in accordance with any one of Claims 1 to 3, characterised in that,
the step size of the image areas (2) is smaller than or equal to the associated width, and the pixels contained in the image areas are entered into the calculation with different weighting.

5. Method for evaluating sides of meat by visual image processing in accordance with any one of Claims 1 to 4, characterised in that,
from the variance of terms within the image areas (2), a confidence measurement is determined, which is allocated to the image areas (2) in further calculations.

6. Method for evaluating sides of meat by visual image processing in accordance with any one of Claims 1 to 5, characterised in that,
optionally in the sixth procedural stage, the second of the two reference points (17), i.e. the intersection (16) with the bacon/MGM boundary (11), is calculated via the acute angular projection of the end-point of the MGM/fat boundary (12) onto the bacon/MGM boundary (11).

7. Method for evaluating sides of meat by visual image processing in accordance with any one of Claims 1 to 6, characterised in that,
further developments and modifications of the two-point method and the calculation specification are taken into account in the sixth procedural stage.

8. Method for evaluating sides of meat by visual image processing in accordance with any one of Claims 1 to 7, characterised in that,
in the sixth procedural stage, the thickness of the bacon and meat is measured at both of the two reference points and entered into the calculation.

9. Method for evaluating sides of meat by visual image processing in accordance with any one of Claims 1 to 8, characterised in that,
the thickness of the bacon and meat is measured at predetermined points between the two reference points and entered into the calculation.

10. Method for evaluating sides of meat by visual image processing in accordance with any one of Claims 1 to 9, characterised in that,
for the line contours (10), characteristic curve parameters are calculated and stored in the memory.

11. Method for evaluating sides of meat by visual image processing in accordance with Claim 10, characterised in that,
an evaluation of the side of meat is carried out and stored via an evaluation function based on the curve parameters for the meat colour calculated in given image areas (2) and an additional weight indication.

12. Method for evaluating sides of meat by visual image processing in accordance with Claim 10, characterised in that,
the evaluation function is verified through reference measurements and/or artificial-intelligence systems.

13. Method for evaluating sides of meat by visual image processing in accordance with any one of Claims 1 to 12, characterised in that,
identification information regarding the side of meat additionally contained in the recorded colour image (1) is selected, read and placed in a memory.

14. Method for evaluating sides of meat by visual image processing in accordance with any one of Claims 1 to 13, characterised in that,
the information is stored in a central memory by means of data transfer.

15. Method for evaluating sides of meat by visual image processing in accordance with Claim 14, characterised in that,
the evaluation function and basic settings can be monitored and/or up-dated via a remote control unit.

## Revendications

1. Procédé d'évaluation de demi-carcasses par traitement optique d'image, selon lequel, on exploite une image en couleur (1), optique, prise par un capteur optique et correspondant à des parties d'une demi-carcasse, en effectuant un traitement totalement automatique assisté par ordinateur,
caractérisé en ce qu'
• on utilise pour l'évaluation, exclusivement la région lombaire élargie de la moitié de la carcasse, accessible optiquement de l'extérieur,
• on calcule pour chaque zone d'image (2), des termes formés par les différents signaux de couleur des pixels,
• dans une première étape, on saisit l'image partielle de la région lombaire élargie de la demi-carcasse et on mémorise électroniquement,
• dans une deuxième étape, on fixe la zone d'exploitation (3) après l'analyse des termes calculés pour chaque zone d'image (2) par l'intermédiaire de l'image partielle,
• dans une troisième étape, on détermine et on mémorise les points de contour (9) par l'analyse des valeurs extrêmes des différences entre les termes calculés pour chaque zone d'image (2) par l'intermédiaire de la zone d'exploitation (3),
• dans une quatrième étape, on calcule et on mémorise les contours linéaires (4) par lissage des bords des zones d'image de contour,
• dans une cinquième étape, on attribue à des lignes de contour (10), la limite de contour extérieure arrière (6), la limite lard/MGM (Musculus-Gluteus-Medius) (11), la limite MGM/graisse (12) et le canal médullaire (13) et on mémorise,
• dans une sixième étape, en fonction des lignes de contour (10), on classe la demi-carcasse selon le procédé ZP (procédé à deux points) en ce qu'au premier point du procédé ZP (15), on calcule l'épaisseur minimale de lard (14) et au deuxième point du procédé ZP (17) (c'est-à-dire au point d'intersection (16) de la limite lard /MGM (11) et de la limite MGM/graisse (12), on calcule l'épaisseur de muscle (18) jusqu'au canal médullaire (13).

2. Procédé d'évaluation de demi-carcasses par traitement optique d'image selon la revendication 1,
caractérisé en ce qu'
on réalise une évaluation sûre pour l'ensemble de la plage de température allant de la carcasse chaude jusqu'à la carcasse froide.

3. Procédé d'évaluation de demi-carcasses par traitement optique d'image selon l'une quelconque des revendications 1 et 2,
caractérisé en ce qu'
avec le procédé ZP, normalisé de manière spéciale à cet effet, on évalue des demi-carcasses de porc.

4. Procédé d'évaluation de demi-carcasses par traitement optique d'image selon l'une quelconque des revendications 1 à 3,
caractérisé en ce que
la longueur de pas des zones d'image (2) est inférieure ou égale à la largeur correspondante et les pixels contenus dans les zones d'image sont utilisés dans le calcul avec des pondérations différentes.

5. Procédé d'évaluation de demi-carcasses par traitement optique d'image selon l'une quelconque des revendications 1 à 4,
caractérisé en ce qu'
à partir de la variance des termes à l'intérieur des zones d'image (2), on définit une mesure de confiance attribuée aux zones d'image (2) pour les autres calculs.

6. Procédé d'évaluation de demi-carcasses par traitement optique d'image selon l'une quelconque des revendications 1 à 5,
caractérisé en ce qu'
en option à la sixième étape du procédé, on calcule le deuxième point du procédé ZP (17), c'est-à-dire que l'on calcule le point d'intersection (16) avec la limite lard/MGM (11) par projection à angle aigu de l'extrémité de la limite MGM/ graisse (12) sur la limite lard/MGM (11).

7. Procédé d'évaluation de demi-carcasses par traitement optique d'image selon l'une quelconque des revendications 1 à 6,
caractérisé en ce que
dans la sixième étape de procédé, on tient compte des développements ultérieurs et des modifications du procédé ZP et de la prescription de calcul.

8. Procédé d'évaluation de demi-carcasses par traitement optique d'image selon l'une quelconque des revendications 1 à 7,
caractérisé en ce que
dans la sixième étape de procédé, on détermine l'épaisseur de lard et de viande aux deux points du procédé (ZP) et on les intègre dans le calcul.

9. Procédé d'évaluation de demi-carcasses par traitement optique d'image selon l'une quelconque des revendications 1 à 8,
caractérisé en ce qu'
on définit l'épaisseur de lard et de viande en différents points de support entre les points du procédé ZP et on l'introduit dans le calcul.

10. Procédé d'évaluation de demi-carcasses par traitement optique d'image selon l'une quelconque des revendications 1 à 9,
caractérisé en ce qu'
on calcule les paramètres de la courbe caractéristique des lignes de contour (10) et on mémorise.

11. Procédé d'évaluation de demi-carcasses par traitement optique d'image selon la revendication 10,
caractérisé en ce qu'
on évalue les demi-carcasses par une fonction d'évaluation à l'aide des paramètres de courbe, de la couleur de la viande calculée dans des zones d'image déterminées (2) et d'une indication supplémentaire de poids, et on mémorise.

12. Procédé d'évaluation de demi-carcasses par traitement optique d'image selon la revendication 10,
caractérisé en ce que
la fonction d'évaluation est vérifiée par des mesures de référence et/ou par des systèmes (KI) d'intelligence artificielle.

13. Procédé d'évaluation de demi-carcasses par traitement optique d'image selon les revendications 1 à 12,
caractérisé en ce qu'
on sélectionne dans l'image couleur (1) prise, des informations d'identification supplémentaires relatives aux demi-carcasses, on les extrait et on les mémorise.

14. Procédé d'évaluation de demi-carcasses par traitement optique d'image selon l'une quelconque des revendications 1 à 13,
caractérisé en ce que
la mémorisation se fait par transmission de données à une mémoire centrale.

15. Procédé d'évaluation de demi-carcasses par traitement optique de l'image selon la revendication 14,
caractérisé en ce que
la fonction d'évaluation et les réglages de base sont surveillés et/ou ou mis à jour par une commande à distance.
